# EUROPEAN PATENT APPLICATION

(11) **EP 2 737 904 A2**
(43) Date of publication of application: **04.06.2014**
(21) Application number: 14000741.0
(22) Date of filing: 11.02.2008
(51) Int. Cl.: A61K 38/18, A61K 31/00, A61K 31/05, A61K 31/335, A61K 31/35, A61K 31/365, A61K 31/395, A61K 31/4015, A61K 45/00, A61P 25/00, A61P 25/28

(54) **Therapeutic effects of bryostatins, bryologs, and other related substances on ischemia/stroke-induced memory impairment and brain injury**

(30) Priority: 09.02.2007 US 900339 P; 24.05.2007 US 924662 P
(62) Divisional of application: 08725396.9
(71) Applicant: Blanchette Rockefeller Neurosciences Institute, Morgantown, WV 26506 (US)
(72) Inventor: Alkon, Daniel, Bethesda Maryland 20817 (US); Sun, Miao-Kun, Gaithersburg Maryland 20879 (US)
(74) Representative: Kador & Partner

(57) **Abstract**

The invention provides for the use of protein kinase activators or boosters of nerve growth factor (NGF), brain-derived neurotrophic factor (BDNF) or other neurotrophic factors to treat stroke. Specifically, the present invention provides methods of treating stroke comprising the steps of identifying a subject having suffered a stroke and administering to said subject an amount of a pharmaceutical composition comprising a protein kinase C (PKC) activator or 4-methylcatechol acetic acid (MCBA) and a pharmaceutically acceptable carrier effective to treat at least one symptom of stroke.

## Description

This application claims benefit to U. S. Provisional Application Serial No. 60/900,339, filed on February 9, 2007 and U. S. Provisional Application Serial No. 60/924,662, filed on May 24, 2007, all of which are hereby incorporated herein by reference in their entireties.

### FIELD OF THE INVENTION

The present invention relates to the treatment of stroke with compounds that activate protein kinase C (PKC) or boost nerve growth factor (NGF), brain-derived neurotrophic factor (BDNF) or other neurotrophic factors.

### BACKGROUND OF THE INVENTION

### A. Stroke

A stroke, also known as cerebrovascular accident (CVA), is an acute neurological injury in which the blood supply to a part of the brain is interrupted. Blood supply to the brain may be interrupted in several ways, including occlusion (ischemic, embolic or thrombotic stroke) or blood-vessel rupture (hemorrhagic stroke). A stroke involves the sudden loss of neuronal function due to disturbance in cerebral perfusion. This disturbance in perfusion is commonly arterial, but can be venous.

The part of the brain with disturbed perfusion no longer receives adequate oxygen. This initiates the ischemic cascade which causes brain cells to die or be seriously damaged, impairing local brain function. Stroke is a medical emergency and can cause permanent neurologic damage or even death if not promptly diagnosed and treated. It is the third leading cause of death and the leading cause of adult disability in the United States and industrialized European nations. On average, a stroke occurs every 45 seconds and someone dies every 3 minutes. Of every 5 deaths from stroke, 2 occur in men and 3 in women.

Despite the medical emergency and the multiple agents that have been shown to be effective in arresting the pathological processes of cerebral ischemia in preclinical studies, thromobolytic therapy using rTPA is currently the only option available for the treatment of ischemic stroke. The treatment is designed to achieve early arterial recanalization, which is time-dependent (within 3 hours after the event to be effective). The effectiveness of rTPA and other potential agents for arresting infarct development, depends on early administration or even before the ischemic event, if possible. The narrow therapeutic time window in treating ischemic stroke leads to about only 5% of candidate patients receiving effective intravenous thrombolytic therapy.

Significant brain injury occurs in ischemic stroke after the immediate ischemic event. The "delayed" brain injury and cell death in cerebral ischemia/stroke is a well-established phenomenon, representing a therapeutic opportunity. Neurons in the infarction core of focal, severe stroke are immediately dead and cannot be saved by pharmacologic intervention. The ischemic penumbra, consisting of the brain tissue around the core in focal ischemic stroke, and the sensitive neurons/network in global cerebral ischemia, however, are maintained by a diminished blood supply. The damage to this penumbral brain tissue occurs in a "delayed" manner, starting 4-6 hours as the second phase or days and weeks later as the the so-called third phase, after cerebral ischemia/stroke. After an about 15 minute cerebral ischemia, for example, the hippocampal CA1 pyramidal cells start to degenerate within 2-3 days, and reach the maximal extent of cell death a week after the ischemic event. The sensitive neuronal structures in global cerebral ischemia and the ischemic penumbra are "at-risk" tissues. Their salvage through intervention or further damage in the subsequent days or weeks determine dramatic differences in long-term disability.

The present invention provides a new therapeutic strategy comprising the transient, periodic or chronic administration of a PKC activator, other compounds and combinations thereof, to a subject suffering from cerebral ischemia/stroke over a broader therapeutic window such as from within hours to days to weeks, after the ischemic event.

### B. Protein Kinase C

PKC has been identified as one of the largest gene families of non-receptor serine-threonine protein kinases. Since the discovery of PKC in the early eighties by Nishizuka and coworkers (Kikkawa et al. (1982) J Biol. Chem. 257: 13341), and its identification as a major receptor for phorbol esters (Ashendel et al. (1983) Cancer Res., 43: 4333), a multitude of physiological signaling mechanisms have been ascribed to this enzyme. The intense interest in PKC stems from its unique ability to be activated *in vitro* by calcium and diacylglycerol (and its phorbol ester mimetics), an effector whose formation is coupled to phospholipid turnover by the action of growth and differentiation factors.

The activation of PKC has been shown to improve learning and memory. (U.S. Patent Application Serial Nos. PCT/US02/13784; PCT/US03/07102; 60/287,721; 60/362,081; 10/172,005; and 10/476,459; each incorporated herein by reference in its entirety). Prior to the present disclosure, however, the PKC-mediated improvement of learning and memory has not been recognized as a mechanism for the treatment of post-stroke memory deficits and brain injury. Also, the PKC activators disclosed herein, specifically those compounds that improve learning and memory, were not recognized as possessing brain function-restoring activity after cerebral ischemia/stroke.

Stroke therapy has historically been limited to few treatment options available. The only drug therapy currently available, for instance, consists of antithrombotics (thrombolytic therapy; such as intravenous injections of tissue plasminogen activator), which have to be administered within 3 hours of the ischemic event. Although many types of potential neuroprotectants have been tested in clinical trials, none has been approved for clinical use, because of ineffectiveness especially when used post-stroke or associated toxicity. The compounds presented in this invention disclosure were effective when the treatment was started 24 hours after the ischemia in the animal model at doses that have already been demonstrated to be well tolerated in humans (the bryostatin-1 doses).Compounds that target the protein kinase C (PKC) such as bryostatin-1, a direct PKC activator, and methylcatechol diacetic acid, a derivative of methylcatechol, an enhancer or means of activating or mobilizing nerve growth factor (NGF), brain-derived neurotrophic factor (BDNF) or other neurotrophic factors, which is perhaps one of the PKC targets, have been found to have therapeutic value against brain injury and memory impairment induced with cerebral ischemia in rats (an animal stroke model). The development of these substances as therapeutic in the treatment of stroke is provided by this invention.

### SUMMARY OF THE INVENTION

The present invention provides methods of treating stroke comprising the steps of identifying a subject having suffered a stroke and administering to said subject an amount of a pharmaceutical composition comprising a protein kinase C (PKC) activator or 4-methylcatechol acetic acid (MCBA) and a pharmaceutically acceptable carrier effective to treat at least one symptom of stroke.

In one embodiment, the PKC activator is FGF-18, a macrocyclic lactone, a benzolactam, a pyrrolidinone, or a combination thereof. In a preferred embodiment, the macrocyclic lactone is a bryostatin or neristatin. In another embodiment, the neristatin is neristatin-1. In another embodiment, the bryostatin is bryostatin-1, 2, 3, 4, 5, 6, 7, 8, 9, 10, 11, 12, 13, 14, 15, 16, 17 or 18. More preferably, the bryostatin is bryostatin-1.

In another preferred embodiment, the pharmaceutical composition comprises 4-methylcatechol acetic acid (MCBA), other derivatives of methylcatechol, or a brain derived neurotrophic factor. MCBA and other derivatives of methylcatechol activate or upregulate nerve growth factor (NGF), brain derived neurotrophic factor (BDNF) or other neurotrophic factors. NGF activates, upregulates or enhances the activity of PKC which in turn upregulates, activates or enhances NGF.

In one embodiment, administration of the pharmaceutical compositions of the present invention is initiated within 1, 2, 3, 4, 5, 6, 7, 8, 9, 10, 11, 12, 13, or 14 days of said stroke. In another embodiment, said administration is initiated between 1 and 2 days, 1 and 3 days, 1 and 4 days, 1 and 5 or 1 and 7 days of said stroke. In another embodiment, the administration of the pharmaceutical compositions of the present invention is initiated within 1, 2, 3, 4, 5, 6, 7, 8, 9, 10, 11, 12, 13, 14, 15, 16, 17, 18, 19, 20, 21, 22, 23, or 24 hours of said stroke. In yet another embodiment, the administration of the pharmaceutical compositions of the present invention is initiated between 1 and 3, 1 and 5, 1 and 10, 1 and 24, 3 and 5, 3 and 10, 3 and 24, 5 and 10, 5 and 24, or 10 and 24 hours after said stroke. In yet another embodiment, the administration of the pharmaceutical compositions of the present invention is initiated after 3, 4, 5, 6, 7, 8, 9, 10, 11, 12, 13, 14, 15, 16, 17, 18, 19, 20, 21, 22, 23, or 24 hours after said stroke/ischemic event. In yet another embodiment, the administration of the pharmaceutical compositions of the present invention is initiated after 1, 2, 3, 4, 5, 6, 7, 8, 9, 10, 11, 12, 13, 14, 15, 16, 17, 18, 19, 20, or 21 days after said stroke/ischemic event.

In one embodiment, treatment comprising the administration of the pharmaceutical compositions of the present invention is continued for a duration of about 1, 2, 3, 4, 5, 6, 7, 8, 9, 10, 11, or 12 weeks.

### BRIEF DESCRIPTION OF THE FIGURE

**Figure 1** depicts a spatial water maze performance of rats over training trials. Data are shown as means ± SEM. Bry, bryostatin-1; Isch, cerebral ischemia; MCDA, 4-methylcatechol-diacetic acid.
**Figure 2** depicts target quadrant ratio during probe test. Bry, bryostatin-1; Isch, ischemia; MCDA, 4-methylcatechol-diacetic acid *: *p* < 0.05. NS: *p* > 0.05.

### DETAILED DESCRIPTION OF THE INVENTION

### A. Definitions

As used herein, "administration" of a composition includes any route of administration, including oral subcutaneous, intraperitoneal, and intramuscular.

As used herein, "an effective amount" is an amount sufficient to reduce one or more symptoms associated with a stroke.

As used herein, "protein kinase C activator" or "PKC activator" means a substance that increases the rate of the reaction catalyzed by protein kinase C by binding to the protein kinase C.

As used herein, the term "subject" means a mammal.

As used herein, the term "pharmaceutically acceptable carrier" means a chemical composition with which the active ingredient may be combined and which, following the combination, can be used to administer the active ingredient to a subject. As used herein, the term "physiologically acceptable" ester or salt means an ester or salt form of the active ingredient which is compatible with any other ingredients of the pharmaceutical composition, which is not deleterious to the subject to which the composition is to be administered.

As used herein, "pharmaceutically acceptable carrier" also includes, but is not limited to, one or more of the following: excipients; surface active agents; dispersing agents; inert diluents; granulating and disintegrating agents; binding agents; lubricating agents; sweetening agents; flavoring agents; coloring agents; preservatives; physiologically degradable compositions such as gelatin; aqueous vehicles and solvents; oily vehicles and solvents; suspending agents; dispersing or wetting agents; emulsifying agents, demulcents; buffers; salts; thickening agents; fillers; emulsifying agents; antioxidants; antibiotics; antifungal agents; stabilizing agents; and pharmaceutically acceptable polymeric or hydrophobic materials. Other "additional ingredients" which may be included in the pharmaceutical compositions of the invention are known in the art and described, for example in Genaro, ed., 1985, Remington's Pharmaceutical Sciences, Mack Publishing Co., Easton, Pa., which is incorporated herein by reference.

The formulations of the pharmaceutical compositions described herein may be prepared by any method known or hereafter developed in the art of pharmacology. In general, such preparatory methods include the step of bringing the active ingredient into association with a carrier or one or more other accessory ingredients, and then, if necessary or desirable, shaping or packaging the product into a desired single- or multi-dose unit.

Although the descriptions of pharmaceutical compositions provided herein are principally directed to pharmaceutical compositions which are suitable for ethical administration to humans, it will be understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and perform such modification with merely ordinary, if any, experimentation. Subjects to which administration of the pharmaceutical compositions of the invention is contemplated include, but are not limited to, humans and other primates, and other mammals.

Despite progress toward the development of new therapeutic agents and availability of several animal models, there is still a pressing need for improved animal models for screening

### B. Protein Kinase C (PKC)

The PKC gene family consists presently of 11 genes which are divided into four subgroups: 1) classical PKCα, β₁, β₂ (β₁ and β₂ are alternatively spliced forms of the same gene) and γ, 2) novel PKCδ, ε, η, and θ, 3) atypical PKCζ, λ, η and *i* and 4) PKC µ. PKC µ resembles the novel PKC isoforms but differs by having a putative transmembrane domain (reviewed by Blohe et al. (1994) Cancer Metast. Rev. 13: 411; Ilug et al. (1993) Biochem J. 291: 329; Kikkawa et al (1989) Ann. Rev. Biochem. 58: 31). The α, β₁, β₂ and γ isoforms are C²⁺, phospholipid and diacylglycerol-dependent and represent the classical isoforms of PKC, whereas the other isoforms are activated by phospholipid and diacylglycerol but are not dependent on Ca²⁺. All isoforms encompass 5 variable (V1-V5) regions, and the α, β and γ isoforms contain four (C1-C4) structural domains which are highly conserved. All isoforms except PKC α, β and γ lack the C2 domain, the λ η and isoforms also lack nine of two cysteine-rich zinc finger domains in C1 to which diacylglycerol binds. The C1 domain also contains the pseudosubstrate sequence which is highly conserved among all isoforms, and which serves an autoregulartory function by blocking the substrate-binding site to produce an inactive conformation of the enzyme (House et al. (1987) Science 238, 1726).

Because of these structural features, diverse PKC isoforms are thought to have highly specialized roles in signal transduction in response to physiological stimuli (Nishizuka (1989) Cancer 10: 1892), as well as in neoplastic transformation and differentiation (Glazer (1994) Protein Kinase C, J.F. Kuo, ed., Oxford U. Press at pages 171-198). For a discussion of known PKC modulators see PCT/US97/08141, U.S. Patent Nos. 5,652,232; 6,080,784; 5,891,906; 5,962,498; 5,955,501; 5,891,870 and 5,962,504 (each incorporated herein by reference in its entirety).

There is increasing evidence that the individual PKC isozymes play significant roles in biological processes which provide the basis for pharmacological exploitation. One is the design of specific (preferably, isozyme specific) activators of PKC. This approach is complicated by the fact that the catalytic domain is not the domain primarily responsible for the isozyme specificity of PKC. These may provide a way to override the effect of other signal transduction pathways with opposite biological effects. Alternatively, by inducing down-regulation of PKC after acute activation, PKC activators may cause long term antagonism. Bryostatin is currently in clinical trials as an anti-cancer agent. The bryostatins are known to bind to the regulatory domain of PKC and to activate the enzyme. Bryostatins are examples of isozyme-selective activators of PKC. (see for example WO 97/43268; incorporated herein by reference in its entirety). For a discussion of known PKC modulators see PCT/US97/08141, U.S. Patent Nos. 5,652,232; 6,043,270; 6,080,784; 5,891,906; 5,962,498; 5,955,501; 5,891,870 and 5,962,504 (each of which is incorporated herein by reference in its entirety).

Several classes of PKC activators have been identified. Phorbol esters, however, are not suitable compounds for eventual drug development because of their tumor promotion activity, (Ibarreta et al. (1999) Neuro Report 10(5&6): 1035-40). Of particular interest are macrocyclic lactones (i.e. bryostatin class and neristatin class) that act to stimulate PKC. Of the bryostatin class compounds., bryostatin-1 has been shown to activate PKC and proven to be devoid of tumor promotion activity. Bryostatin-1, as a PKC activator, is also particularly useful since the dose response curve of bryostatin-1. is biphasic. Additionally, bryostatin-1 demonstrates differential regulation of PKC isozymes, including PKCα, PKCδ and PKCε. Bryostatin-1 has undergone toxicity and safety studies in animals and humans and is actively investigated as an anti-cancer agent. Bryostatin-1's use in the studies has determined that the main adverse reaction in humans is myalgia. One example of an effective dose is 40 µg/m² per week by intravenous injection.

Macrocyclic lactones, and particularly bryostatin-1 is described in U.S. Patent 4,560,774 (incorporated herein by reference in its entirety). Macrocyclic lactones and their derivatives are described elsewhere in U.S. Patent 6,187,568, U.S. Patent 6,043,270, U.S. Patent 5,393,897, U.S. Patent 5,072,004, U.S. Patent 5,196,447, U.S. Patent 4,833,257, and U.S. Patent 4,611,066 (incorporated herein by reference in its entirety). The above patents describe various compounds and various uses for macrocyclic lactones including their use as an antiinflammatory or anti-tumor agent. (Szallasi et al. (1994) Journal of Biological Chemistry 269(3): 2118-24; Zhang et al. (1996) Caner Research 56: 802-808; Hennings et al. (1987) Carcinogenesis 8(9): 1343-1346; Varterasian et al. (2000) Clinical Cancer Research 6: 825-828; Mutter et al. (2000) Bioorganic & Medicinal Chemistry 8: 1841-1860)(each incorporated herein by reference in its entirety).

As will also be appreciated by one of ordinary skill in the art, macrocyclic lactone compounds and their derivatives, particularly the bryostatin class, are amenable to combinatorial synthetic techniques and thus libraries of the compounds can be generated to optimize pharmacological parameters, including, but not limited to efficacy and safety of the compositions. Additionally, these libraries can be assayed to determine those members that preferably modulate α-secretase and/or PKC.

Combinatorial libraries high throughput screening of natural products and fermentation broths has resulted in the discovery of several new drugs. At present, generation and screening of chemical diversity is being utilized extensively as a major technique for the discovery of lead compounds, and this is certainly a major fundamental advance in the area of drug discovery. Additionally, even after a "lead" compound has been identified, combinatorial techniques provide for a valuable tool for the optimization of desired biological activity. As will be appreciated, the subject reaction readily lend themselves to the creation of combinatorial libraries of compounds for the screening of pharmaceutical, or other biological or medicallyrelated activity or material-related qualities. A combinatorial library for the purposes of the present invention is a mixture of chemically related compounds, which may be screened together for a desired property; said libraries may be in solution or covalently linked to a solid support. The preparation of many related compounds in a single reaction greatly reduces and simplifies the number of screening processes that need to be carried out. Screening for the appropriate biological property may be done by conventional methods. Thus, the present invention also provides methods for determining the ability of one or more inventive compounds to bind to effectively modulate α-secretase and/or PKC.

A variety of techniques are available in the art for generating combinatorial libraries described below, but it will be understood that the present invention is not intended to be limited by the foregoing examples and descriptions. (See, for example, Blondelle et al. (1995) Trends Anal. Chem. 14: 83; U.S. Patents 5,359,115; 5,362,899; U.S. 5,288,514: PCT publication WO 94/08051; Chen et al. (1994) JACCS 16:266 1: Kerr et al. (1993) JACCS I 1 5:252; PCT publications W092/10092, W093/09668; W091/07087; and W093/20242; each of which is incorporated herein by reference). Accordingly, a variety of libraries on the order of about 16 to 1,000,000 or more diversomers can be synthesized and screened for a particular activity or property.

Analogs of bryostatin, commonly referred to as bryologs, are one particular class of PKC activators that are suitable for use in the methods of the present invention. The following Table summarizes structural characteristics of several bryologs, demonstrating that bryologs vary greatly in their affinity for PKC (from 0.25 nM to 10 µM). Structurally, they are all similar. While bryostatin-1 has two pyran rings and one 6-membered cyclic acetal, in most bryologs one of the pyrans of bryostatin-1 is replaced with a second 6-membered acetal ring. This modification reduces the stability of bryologs, relative to bryostatin-1, for example, in both strong acid or base, but has little significance at physiological pH. Bryologs also have a lower molecular weight (ranging from about 600 to 755), as compared to bryostatin-1 (988), a property which facilitates transport across the blood-brain barrier.

| **Name** | **PKC Affin (nM)** | **MW** | **Description** |
|---|---|---|---|
| Bryostatin 1 | 1.35 | 988 | 2 pyran + 1 cyclic acetal + macrocycle |
| Analog 1 | 0.25 | 737 | 1 pyran + 2 cyclic acetal + macrocycle |
| Analog 2 | 6.50 | 723 | 1 pyran + 2 cyclic acetal + macrocycle |
| Analog 7a | - | 642 | 1 pyran + 2 cyclic acetals + macrocycle |
| Analog 7b | 297 | 711 | 1 pyran + 2 cyclic acetals + macrocycle |
| Analog 7c | 3.4 | 726 | 1 pyran + 2 cyclic acetals + macrocycle |
| Analog 7d | 10000 | 745 | 1 pyran + 2 cyclic acetals + macrocycle, acetylated |
| Analog 8 | 8.3 | 754 | 2 cyclic acetals + macrocycle |
| Analog 9 | 10000 | 599 | 2 cyclic acetals |

Analog 1 (Wender et al. (2004) Curr Drug Discov Technol. 1: 1; Wender et al. (1998) Proc Natl Acad Sci U S A 95: 6624; Wender et al. (2002) Am Chem Soc. 124: 13648 (each incorporated herein by reference in their entireties)) possesses the highest affinity for PKC. This bryolog is about100 times more potent than bryostatin-1. Only Analog 1 exhibits a higher affinity for PKC than bryostatin. Analog 2, which lacks the A ring of bryostatin-1 is the simplest analog that maintains high affinity for PKC. In addition to the active bryologs, Analog 7d, which is acetylated at position 26, hasvirtually no affinity for PKC.

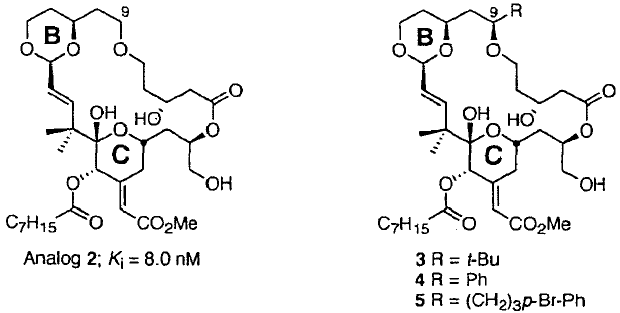

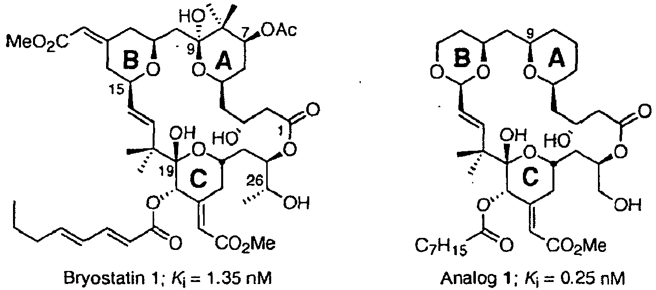

B-ring bryologs are also suitable for use in the methods of the present invention. These synthetic bryologs have affinities in the low nanomolar range (Wender et al. (2006) Org Lett. 8: 5299 (incorporated herein by reference in its entirety)). The B-ring bryologs have the advantage of being completely synthetic, and do not require purification from a natural source.

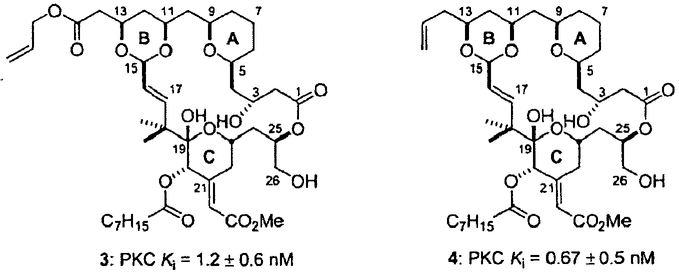

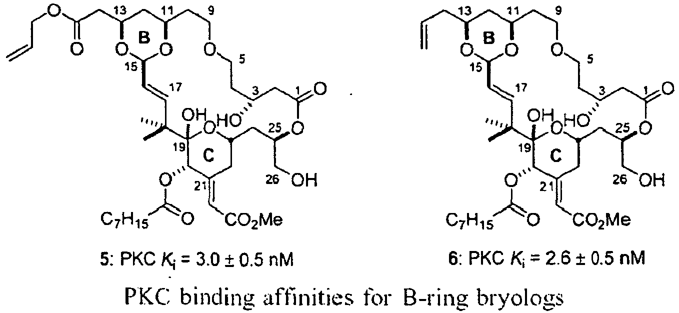

A third class of suitable bryostatin analogs is the A-ring bryologs. These bryologs have slightly lower affinity for PKC than bryostatin I (6.5, 2.3, and 1.9 nM for bryologs 3, 4, and 5, respectively) but have a lower molecular weight.

A number of derivatives of diacylglycerol (DAG) bind to and activate protein kinase C (Niedel et al. (1983) Proc. Natl. Acad. Sci. USA 80: 36; Mori et al. (1982) J. Biochem (Tokyo) 91: 427; Kaibuchi et al. (1983) J. Biol. Chem. 258: 6701). However, DAG and DAG derivatives are of limited value as drugs. Activation of PKC by diacylglycerols is transient, because they are rapidly metabolized by diacylglycerol kinase and lipase (Bishop et al.. (1986) J. Biol. Chem. 261: 6993; Chung et al. (1993) Am. J. Physiol. 265: C927; incorporated herein by reference in their entireties). The fatty acid substitution determines the strength of activation. Diacylglycerols having an unsaturated fatty acid are most active. The stereoisomeric configuration is also critical. Fatty acids with a 1,2-sn configuration are active, while 2,3-sn-diacylglycerols and 1,3-diacylglycerols do not bind to PKC. Cis-unsaturated fatty acids are synergistic with diacylglycerols. In one embodiment of the present invention, the term "PKC activator" expressly excludes DAG or DAG derivatives, such as phorbol esters.

Isoprenoids are PKC activators suitable for use in the methods of the present invention. Farnesyl thiotriazole, for example, is a synthetic isoprenoid that activates PKC with a Kd of 2.5 µM. Farnesyl thiotriazole, for example, is equipotent with dioleoylglycerol (Gilbert et al. (1995) Biochemistry 34: 3916; incorporated herein by reference in its entirety), but does not possess hydrolyzable esters of fatty acids. Farnesyl thiotriazole and related compounds represent a stable, persistent PKC activator. Because of its low MW (305.5) and absence of charged groups, farnesyl thiotriazole would readily cross the blood-brain barrier.

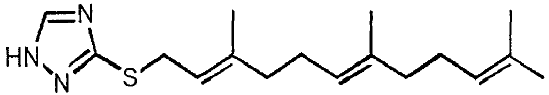

Octylindolactam V is a non-phorbol protein kinase C activator related to teleocidin. The advantages of octylindolactam V, specifically the (-)-enantiomer, include greater metabolic stability, high potency (Fujiki et al. (1987) Adv. Cancer Res. 49: 223; Collins et al. (1982) Biochem. Biophys. Res. Commun. 104: 1159; each incorporated herein by reference in its entirety)(EC50 = 29nM) and low molecular weight that facilitates transport across the blood brain barrier.

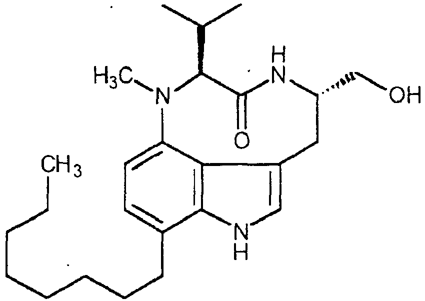

Gnidimacrin is a daphnane-type diterpene that displays potent antitumor activity at concentrations of 0.1 - 1 nM against murine leukemias and solid tumors. It acts as a PKC activator at a concentration of ≈3 nM in K562 cells, and regulates cell cycle progression at the G1/S phase through the suppression of Cdc25A and subsequent inhibition of cyclin dependent kinase 2 (Cdk2) (100% inhibition achieved at 5 ng/ml). Gnidimacrin is a heterocyclic natural product similar to bryostatin, but somewhat smaller (MW = 774.9).

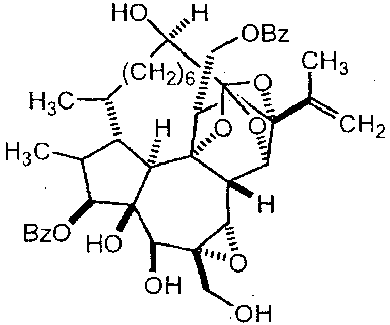

Iripallidal is a bicyclic triterpenoid isolated from Iris pallida. Iripallidal displays anti-proliferative activity in a NCI 60 cell line screen with GI50 (concentration required to inhibit growth by 50%) values from micromolar to nanomolar range. It binds to PKCα with high affinity (Ki = 75.6 nM). It induces phosphorylation of ERK1/2 in a RasGRP3-dependent manner. M.W. 486.7. Iripallidal is only about half the size of bryostatin and lacks charged groups.

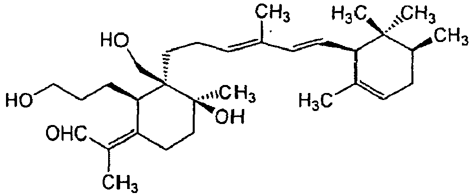

Ingenol is a diterpenoid related to phorbol but possesses much less toxicity. It is derived from the milkweed plant Euphorbia peplus. Ingenol 3,20-dibenzoate, for example, competes with [3H]phorbol dibutyrate for binding to PKC (Ki for binding=240 nM) (Winkler et al. (1995) J.Org.Chem. 60: 1381; incorporated herein by reference). Ingenol-3-angelate possesses antitumor activity against squamous cell carcinoma and melanoma when used topically (Ogbourne et al. (2007) Anticancer Drugs. 18: 357; incorporated herein by reference).

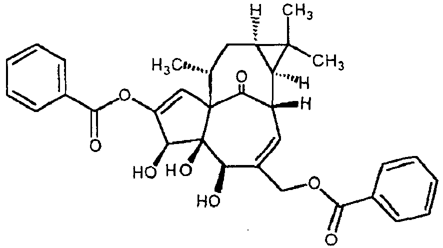

Napthalenesulfonamides, including N-(n-heptyl)-5-chloro-1-naphthalenesulfonamide (SC-10) and N-(6-Phenylhexyl)-5-chloro-1-naphthalenesulfonamide, are members of another class of PKC activators. SC-10 activates PKC in a calcium-dependent manner, using a mechanism similar to that of phosphatidylserine (Ito et al. (1986) Biochemistry 25: 4179; incorporated herein by reference). Naphthalenesulfonamides act by a different mechanism from bryostatin and would be expected to show a synergistic effect with bryostatin or a member of another class of PKC activators. Structurally, naphthalenesulfonamides are similar to the calmodulin (CaM) antagonist W-7, but are reported to have no effect on CaM kinase.

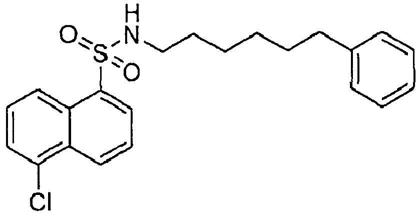

The linoleic acid derivative DCP-LA (2-[(2-pentylcyclopropyl)methyl] cyclopropaneoctanoic acid) is one of the few known isoform-specific activators of PKC known. DCP-LA selectively activates PKCε with a maximal effect at 100 nM. (Kanno et al. (2006) J. Lipid Res. 47: 1146). Like SC-10, DCP-LA interacts with the phosphatidylserine binding site of PKC, instead of the diacylglycerol binding site.

An alternative approach to activating PKC directly is to increase the levels of the endogenous activator, diacylglycerol. Diacylglycerol kinase inhibitors such as 6-(2-(4-[(4-fluorophenyl)phenylmethylene]-1-piperidinyl)ethyl)-7-methyl-5H-thiazolo[3,2-a]pyrimidin-5-one (R59022) and [3-[2-[4-(bis-(4-fluorophenyl)methylene]piperidin-1-yl)ethyl]-2,3-dihydro-2-thioxo-4(1H)-quinazolinone (R59949) enhance the levels of the endogenous ligand diacylglycerol, thereby producing activation of PKC (Meinhardt et al. (2002) Anti-Cancer Drugs 13: 725).

A variety of growth factors, such as fibroblast growth factor 18 (FGF-18) and insulin growth factor, function through the PKC pathway. FGF-18 expression is upregulated in learning and receptors for insulin growth factor have been implicated in learning. Activation of the PKC signaling pathway by these or other growth factors offers an additional potential means of activating protein kinase C.

Growth factor activators, such as the 4-methyl catechol derivatives, such as 4-methylcatechol acetic acid (MCBA), that stimulate the synthesis and/or activation of growth factors such as NGF and BDNF, also activate PKC as well as convergent pathways responsible for synaptogenesis and/or neuritic branching.

The present compounds can be administered by a variety of routes and in a variety of dosage forms including those for oral, rectal, parenteral (such as subcutaneous, intramuscular and intravenous), epidural, intrathecal, intra-articular, topical and buccal administration. The dose range for adult human beings will depend on a number of factors including the age, weight and condition of the patient and the administration route.

All books, articles, patents or other publications and references are hereby incorporated by reference in their entireties. Reference to any compound herein includes the racemate as well as the single enantiomers.

### EXAMPLES

The following Examples serve to further illustrate the present invention and are not to be construed as limiting its scope in any way.

### EXAMPLE 1: Global Ischemia Model of Stroke

Rats (male, Wistar, 200 - 225g) were randomly divided into 6 groups (8 each) and housed for I week before experimentation. Transient or permanent restriction of cerebral blood flow and oxygen supply results in ischemic stroke. The global ischemia model used to induce vascular memory impairment was two-vessel occlusion combined with a short term systemic hypoxia. Ligation of the bilateral common carotid arteries was performed under anesthesia (pentobarbital, 60 mg/kg, i.p.). After a one-week recovery from the surgery, rats were exposed to 14-min hypoxia (5% oxygen in a glass jar). Control rats (sham operated and vehicle controls) were subjected to the same incision to isolate both common carotid arteries and to 14-min air (in the glass jar). Body temperature was kept at 37-37.5 °C using a heating light source during the surgical procedure and until the animals were fully recovered.

### EXAMPLE 2: Bryostatin and MCDA Treatment

Bryostatin-1 was administered at 20 µg/g/m² (tail i.v., 2 doses/week, for 10 doses), starting 24 hours after the end of the hypoxic event. 4-Methylcatechol-diacetic acid (MCDA, a potential NGF and BDNF booster) was administered at 1.0 mg/kg (i.p., daily for the same 5-week period) in separate groups of rats.

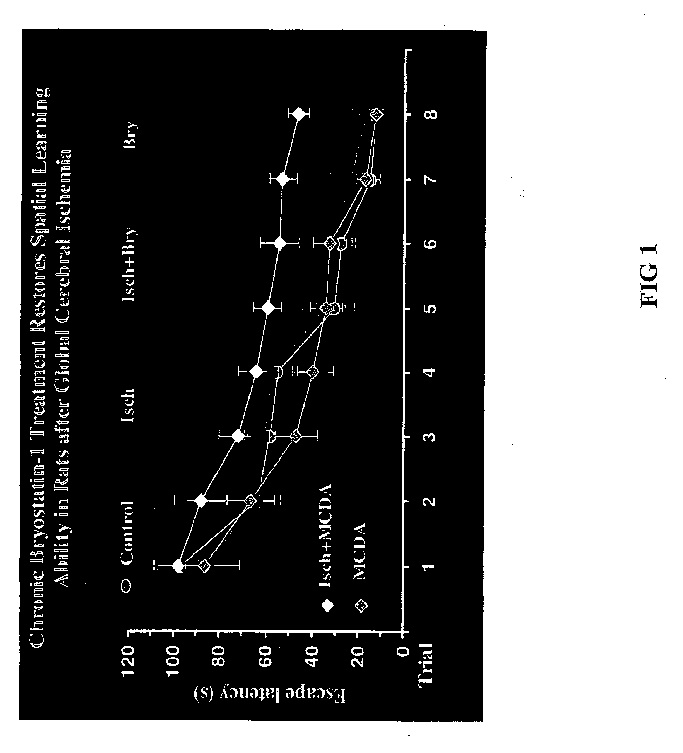

One week after the last bryostatin-1, MCDA, or vehicle administration; rats were trained in the water maze spatial learning task (2 training trials per day for 4 days), followed by a probe test. A visible platform test was given after the probe test. The results are shown in **Figure 1**.

Overall, there was a significant learning difference between the 6 groups (Figure 1; *F*₅,₃₈₃= 27.480, *p* < 0.001; ANOVA). Detailed analysis revealed that the ischemic group did not learn the spatial maze task since there was no significant difference in escape latency over trials (*F*_{7,63} = 0.102, *p* > 0.05), a significantly impaired learning as compared with the control rats (group difference: *F*_{1,127} = 79.751, *p* < 0.001), while the rats in the other 5 groups all learned the task (the ischemic rats with MCDA treatment: *p* < 0.05 and the other 4 groups: *p* < 0.001 over trials). Bryostatin-1 therapy greatly improved the performance (Ischemic group with bryostatin-1 treatment vs. ischemic rats: *F*_{1,127} = 72.782, *p* < 0.001), to the level of performance that did not differ statistically from the control rats (Ischemic group with bryostatin-1 treatment vs. control rats: *F*_{1,127} = 0.001, *p* > 0.05). MCDA treatment also improved the learning of the ischemic rats (ischemia with NCDA treatment vs. ischemic rats: *F*_{1,127} = 15.584, *p* < 0.001) but the difference between the ischemia with MCDA treatment and control rats remained significant after the 5 week treatment (ischemia with NCDA treatment vs. control rats: *F*_{1,127} = 16.618, *p* < 0.001). There were no differences between the control and bryostatin-1-only groups (bryostatin-1 vs. control: *F*_{1,127} = 0.010, *p* > 0.05) and between the control and MCDA-only groups (MCDA vs. control: *F*_{1,127} = 0.272, *p* > 0.05).

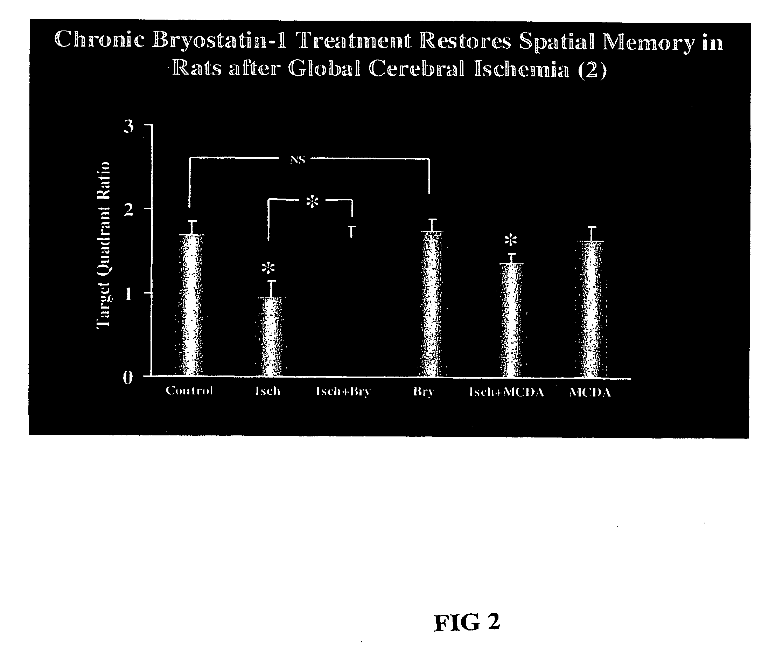

The rats in the ischemic group did not show a target preference in the probe test (F3,31 = 0.096, p > 0.05), while the rats of the other 5 groups all showed a target quadrant preference in the probe test (all p < 0.005). Data were analyzed using target quadrant ratio (dividing the target quadrant distance by the average of the non-target quadrant values during the probe test; Figure 2). There was a significant difference in the target quadrant ratios between the groups (F5,47 = 5.081, p < 0.001). Detailed analysis revealed group differences between the control and ischemic rats (F1,15 = 9.451, p < 0.01), between the ischemic and ischemic with bryostatin-1 treatment (F1,15 = 10.328, p < 0.01), and between the ischemic with MCDA treatment and ischemic rats (F1,15 = 5.623, p < 0.05), but no differences between the control and ischemic rats with bryostatin-1 treatment (F1, 15 = 0.013, p > 0.05), between the ischemic with MCDA treatment and control groups (F1,15 = 2.997, p > 0.05), between the control and bryostatin-1-only rats (F1,15 = 0.064, p > 0.05), and between the control and the MCDA-only rats (F1,15 = 0.0392, p > 0.05). A visible platform test, determined after the probe test revealed no significant difference between the groups (F5,47 = 0.115, p > 0.05), indicating that there were no significant group differences in sensorimotor ability of the rats.

### EXAMPLE 3: Bryostatin Treatment

Global cerebral ischemia/hypoxia was induced in male Wistar rats (225-250 g) by permanently occluding the bilateral common carotid arteries, combined with about 14 minutes of low oxygen (about 5%). Bryostatin- was administered at 15 µg/m² (via a tail vein, 2 doses/week, for 10 doses), starting about 24 hours after the end of the ischemic/hypoxic event. Spatial learning (2 trials/ day for 4 days) and memory (a probe test of 1 minute, 24 hours after the last trial) task was performed 9 days after the last dose. Overall, there was a significant difference between the groups (F3,255 = 31.856, p<0.001) and groups x trials (F21,255 =1.648, p<0.05). Global cerebral ischemia impaired the spatial learning (ischemial vs. sham-operated F1,127 = 79.751, p>0.001). The learning impairment was restored by Bryostatin-1 treatment (Bryostatin-1 + Ischemia vs. Ischemia: F1,127=50.233, p<0.001), while Bryostatin-1 alone did not affect the learning (Bryostatin-1 vs. sham-operated: F1,127 = 2.258, p>0.05; 9 days after the last dose).

In the memory retention test, sham-operated rats showed a target quadrant preference. Such good memory retention was not observed in the ischemic rats, indicating an impaired spatial memory. Bryostatin-1 therapy effectively restored memory retention after ischemia to the level of the sham-operated rats. Bryostatin-1 alone had no significant effects in the target quadrant preference compared with that of the sham-operated control rats. There was a significant difference in the quadrant ratios (calculated by dividing the target quadrant swim distance by the average swim distance in the non-target quadrants; F3,31 = 6.181, p<0.005) between the groups. Detailed analysis revealed significant differences between the ischemic rats and sham-operated control rats (F1,15 = 9.451, p<0.01), between the ischemic rats and ischemic rats with Bryostatin-1 treatment (F1,15 = 10.328, p<0.01), but no significant differences between the ischemic rats with Bryostatin-1 treatment and sham-operated control (F1,15 = 0.0131, p>0.05) and between the sham-operated control rats and Bryostatin-1 alone rats (F1,15 = 0.161, p>0.05). These results demonstrate that the cerebral ischemia/hypoxia produced an impairment of spatial learning and memory, tested about 7 weeks after the ischemic event. The impairment was lasting and not recoverable, during the time frame without appropriate intervention, but restored by chronic Bryostatin-1 treatment, even when the treatment was started 24 hours after the ischemic event, a wide therapeutic time-window.

**In the following clauses, preferred embodiments of the invention are described:**
1. A method of treating stroke comprising the steps of identifying a subject having suffered an ischemic event and administering to said subject an amount of a pharmaceutical composition comprising a protein kinase C (PKC) activator or 4-methylcatechol acetic acid (MCBA), or other derivatives of methylcatechol, and a pharmaceutically acceptable carrier effective to treat at least one symptom of stroke.
2. The method of clause 1, wherein the PKC activator is FGF- 18, a macrocyclic lactone, a benzolactam, a pyrrolidinone, or a combination thereof.
3. The method of clause 2, wherein the macrocyclic lactone is a bryostatin or neristatin.
4. The method of clause 3, wherein the bryostatin is bryostatin-1, 2, 3, 4, 5, 6,7, 8, 9,10,11,12, 13, 14, 15, 16, 17 or 18.
5. The method of clause 4, wherein the bryostatin is bryostatin-1.
6. The method of clause 3, wherein the neristatin is neristatin- 1.
7. The method of clause 1, wherein the pharmaceutical composition comprises 4- methylcatechol acetic acid.
8. The method of clause 1, wherein said administration is initiated within 1 day of said stroke.
9. The method of clause 1, wherein said administration is initiated within 2 days of said stroke.
10. The method of clause 1, wherein said administration is initiated within 3 days of said stroke.
11. The method of clause 1, wherein said administration is initiated between 1 and 2 days of said stroke.
12. The method of clause 1, wherein said administration is initiated between 1 and 3 days of said stroke.
13. The method of clause 1, wherein the treatment is continued for a duration of 1 week.
14. The method of clause 1 wherein the treatment is continued for a duration of 2 weeks.
15. The method of clause 1, wherein the treatment is continued for a duration of 3 weeks.
16. The method of clause 1, wherein the treatment is continued for a duration of 4 weeks.
17. The method of clause 1, wherein the treatment is continued for a duration of 6 week.
18. The method of clause 1 wherein said treatment reverses stroke-induced brain injury.
19. The method of clause 1, wherein said treatment reverses stroke-induced memory impairment.

## Claims

1. A pharmaceutical composition comprising a protein kinase C (PKC) activator or 4-methylcatechol acetic acid (MCBA), or other derivatives of methylcatechol, and a pharmaceutically acceptable carrier for use in a medicament effective to treat at least one symptom of stroke, wherein the PKC activator is selected from the group consisting of FGF-18, a macrocyclic lactone, a benzolactam, a pyrrolidinone, bryologs, diacylglycerol derivatives other than phorbol esters, isoprenoids, daphnane-type diterpenes, bicyclic triterpenoids, naphthalenesulfon-amides, lineolic acid derivatives, diacylglycerol kinase inhibitors, growth factor activators and a combination thereof.

2. The pharmaceutical composition of claim 1, wherein the PKC activator is a macrocyclic lactone.

3. The pharmaceutical composition of claim 2, wherein the macrocyclic lactone is a bryostatin or neristatin.

4. The pharmaceutical composition of claim 3, wherein the bryostatin is bryostatin- 1, 2, 3, 4, 5, 6, 7, 8, 9, 10, 11 , 12, 13, 14, 15, 16, 17 or 18.

5. The pharmaceutical composition of claim 4, wherein the bryostatin is bryostatin- 1.

6. The pharmaceutical composition of claim 3, wherein the neristatin is neristatin- 1.

7. The pharmaceutical composition of claim 1, wherein the pharmaceutical composition comprises 4- methylcatechol acetic acid.

8. The pharmaceutical composition of claim 1, wherein the administration of said pharmaceutical composition is initiated at a time period chosen from within 1 day, within 2 days, within 3 days, between 1 and 2 days, and between 1 and 3 days of said stroke.

9. The pharmaceutical composition of claim 1, wherein the treatment is continued for a duration chosen from 1 week, 2 weeks, 3 weeks, 4 weeks, 5 weeks or 6 weeks.

10. The pharmaceutical composition of claim 1, wherein said treatment reverses stroke- induced brain injury.

11. The pharmaceutical composition of claim 1, wherein said treatment reverses stroke-induced memory impairment.
